# EUROPEAN PATENT APPLICATION

(11) **EP 1 679 060 A1**
(43) Date of publication of application: **12.07.2006**
(21) Application number: 04791950.1
(22) Date of filing: 01.10.2004
(51) Int. Cl.: A61K 8/18, A61K 36/18

(54) **HAIR GROWTH STIMULANT COMPOSITION**

(30) Priority: 09.10.2003 JP 2003350807
(71) Applicant: Advangen, Inc., Ibaraki 3058566 (JP)
(72) Inventor: SUZUKI, S. National Inst. Adv. Ind. Sce. & Tech., Ibaraki 3058566 (JP); TOGASHI, K. National Inst. Adv. Ind. Sce. & Tech., Ibaraki 3058566 (JP); HORI, M. National Inst. Adv. Ind. Sce. & Tech., Ibaraki 3058566 (JP); SUGAI, N. National Inst. Adv. Ind. Sce. & Tech., Ibaraki 3058566 (JP)
(74) Representative: Webber, Philip Michael
(86) International application number: PCT/JP2004/014483
(87) International publication number: WO 2005/034894

(57) **Abstract**

Fibroblast growth factor 5 (FGF-5) has a function of inhibiting hair growth and thereby shifting hair cycle of hair matrix cells from the growing stage to the regression stage. Extracts obtained by extraction preferably from rose fruit, loquat, burnet, bearberry, mate, cat's claw, mugwort, *Uncaria rhynchophylla,* tea and brown algae with water or a solvent other than water have an activity of inhibiting the function of FGF-5.
Therefore, a hair growth stimulant and a cosmetic containing one or more kinds of the above extracts contain ingredients capable of maintaining proliferation of hair matrix cells and directly promoting hair growth, so that they exert excellent hair growth-stimulating and hair-nourishing effects based on the hair cycle control mechanism of FGF-5.

## Description

### TECHNICAL FIELD

The present invention relates to a novel plant extract which inhibits an activity of fibroblast growth factor 5, a hair growth stimulant containing the extract and a cosmetic containing the extract.

### BACKGROUND ART

Most of hair growth-stimulating or hair-nourishing agents hitherto developed aim at promotion of hair growth by suppressing excess sebum secretion that inhibits hair growth or by improving poor blood circulation of the scalp or the like or by suppressing actions of the male hormone. On the other hand, hair growth stimulants derived from plants such as pine extract exist from old times, and they have been used as popular remedies although the essence of their hair growth-stimulating effects is not clear. Thus, a great number of hair growth-stimulating or hair-nourishing agents are present, but any agent that universally exerts effects on thinning of hair or loss of hair does not exist at present, and the individual difference of the effects due to the type of thinning of hair or loss of hair is great.

By the way, hair does not grow constantly at a fixed rate but grows with repeating the growing stage and the resting stage. The cycle of hair growth and falling-out of hair, that is, the cycle from the growing stage to the resting stage (hair cycle) differs among the body parts, and in case of the human hair, the hair cycle differs in every hair. Transforming growth factor (TGF)β that is a substance having influence on the hair cycle of the hair matrix cell performs a function of shifting the hair cycle from the growing stage to the regression stage or the resting stage, and an antagonist that inhibits this function has been found and proposed as a novel hair growth-stimulating ingredient.

The present inventors have found that fibroblast growth factor 5 (also referred to as "FGF-5" hereinafter) which is a cytokine different from the TGFβ also has a biological activity of controlling the hair cycle. The FGF-5 protein belongs to the fibroblast growth factor (FGF) family exhibiting various physiological functions. As a result of studies by the present inventors, it has been elucidated that FGF-5 has a function of inhibiting hair growth and thereby shifting the hair cycle from the growing stage to the regression stage. It has been also demonstrated that FGF-5S, which is a short chain molecule of the FGF-5 and occurs in a large amount in the hair follicles of the growing stage, exhibits an antagonist activity against the FGF-5 and thereby retards shifting of the hair cycle from the growing stage to the regression stage to resultantly promote the hair growth (see non-patent documents 1 to 4).

In the existing circumstances described above, it can be said that needs for hair growth-stimulating or hair-nourishing agents that universally exert effects on thinning of hair or loss of hair are still high and will be high in future, reflecting the society of aged people and high stresses.

Non-patent document 1: Ozawa K., Suzuki S. and Asada *M., J. Biol. Chem.* 273, 29262-29271 (1998)

Non-patent document 2: Suzuki S., Kato T. and Takimoto H., *J. Invest. Dermatol.* 111, 963-972 (1998)

Non-patent document 3: Suzuki S., Ota Y., Ozawa K. and Imamura T., *J. Invest. Dermatol.* 114, 456-463 (2000)

Non-patent document 4: Ota Y., Saitoh Y. and Suzuki *S., Biochem. Biohphys. Res. Commun.* 290, 169-176 (2002)

### DISCLOSURE OF THE INVENTION

During the course of studies on the FGF-5 function, the present inventors have had an idea that a substance having an activity of inhibiting the function can be used as a hair growth stimulant exerting an excellent effect, and they have further studied earnestly in order to find a substance which exerts an action of inhibiting the FGF-5 function of suppressing hair growth or becomes an antagonist against the FGF-5. As a result, the present inventors have found that a specific plant extract serves the purpose, and based on the findings, the present invention has been thus accomplished.

It is an object of the present invention to provide a FGF-5 inhibitor, a hair growth stimulant and a cosmetic each of which contains such a plant extract. That is to say, the object of the invention is to provide a hair growth stimulant and a cosmetic, which exert excellent hair growth-stimulating and hair-nourishing effects based on the hair cycle control mechanism of FGF-5 and are almost free from harmful side effects.

### SUMMARY OF THE INVENTION

The present invention is a plant extract usually obtained by extraction from a plant with water or a solvent other than water and having an activity of inhibiting a function of fibroblast growth factor 5 (FGF-5) .

The plant extract of the invention can be obtained by extraction from a plant belonging to Rosaceae, Ericaceae, Aquifoliaceae, Rubiaceae, Compositae or Phaeophyceae with water or a solvent other than water and has an activity of inhibiting a function of FGF 5.

In the plant extract, the plant is preferably selected from the group consisting of rose fruit, loquat, burnet, bearberry, mate, cat's claw, mugwort and brown algae.

A plant extract obtained by extraction from *Uncaria rhynchophylla* or tea with water or a solvent other than water and having an activity of inhibiting a function of FGF-5 is also included in the present invention.

The present invention is a fibroblast growth factor 5 inhibitor containing one or more kinds of extracts obtained by extraction from rose fruit, loquat, burnet, bearberry, mate, cat's claw, mugwort, *Uncaria rhynchophylla*, tea and brown algae with water or a solvent other than water.

The present invention is a hair growth stimulant containing as active ingredients one or more kinds of the above-mentioned plant extracts which inhibit a function of FGF-5.

The present invention is a cosmetic containing, as active ingredients, one or more kinds of the above-mentioned plant extracts which inhibit a function of FGF-5.

### EFFECT OF THE INVENTION

The plant extracts (extracted essences) of the present invention obtained by extraction from plants or seaweeds have proved to possess an activity of inhibiting a function of FGF-5. The function of FGF-5 is an inhibition of hair growth and thereby shifting the hair cycle of the hair matrix cell from the growing stage to the regression stage.

### DETAILED DESCRIPTION OF THE INVENTION

The present invention is described in detail hereinafter with reference to a plant extract, inhibition of FGF-5, preparation of a plant extract, a hair growth stimulant and a cosmetic.

### Plant extract

The plant extract of the invention can be obtained by extraction from a plant with water or a solvent other than water and has an activity of inhibiting a function of fibroblast growth factor 5 (FGF-5). The plant extract is preferably an extract from a plant selected from the group consisting of plants of Rosaceae, Ericaceae, Aquifoliaceae, Rubiaceae, Compositae, Theaceae and Phaeophyceae, or an extract derived from the extract. The plant used as a raw material for preparing the extract of the invention is particularly preferably selected from the group consisting of rose fruit, loquat, burnet, bearberry, mate, cat's claw, *Uncaria rhynchophylla*, mugwort, tea and brown algae, among the above-described plants.

Rose fruit used in the invention is false fruit or fruit of *Rosa multiflora Thunb.* (botanical name, Rosaceae) that is a liana deciduous shrub naturally growing in Japan or another relative plant (Rosaceae). Loquat (botanical name: *Eriobotrya japonica Lindley)* is a limestone-phile, evergreen tree plant of Rosaceae that grows wild in the Kanto district or westward. Burnet (botanical name: *Sanguisorba officinalis)* is a perennial herbaceous plant of Rosaceae that naturally grows in sunshiny fields and mountains in the whole of Japan. Bearberry (botanical name: *Arctostaphylos uva-ursi(Linne) Sprengel* (Ericaceae)) is an evergreen small shrub of Ericaceae that naturally grows in alpine zones and fields of cold districts in the northern hemisphere. Mate (botanical name: *Ilex Paraguariensis)* is a plant of Aquifoliaceae that naturally grows in the subtropical zones, and cat's claw (botanical name: *Uncaria tomentosa)* is a plant belonging to Uncaria of Rubiaceae that grows wild in the tropical rainy forest regions of the whole world. As a plant belonging to the same Uncaria of Rubiaceae, *Uncaria rhynchophylla* (botanical name) is an evergreen liane. Tea (botanical name: *Camellia sinensis)* is a plant belonging to Theaceae, and mugwort (botanical name: *Artemisia princes)* is a perennial herbaceous plant of Compositae that is generally found in levees and meadows. The seaweeds are particularly preferably brown algae (Class Phaeophyceae).

The term "plant extracts" used herein means various solvent extracts obtained by subjecting raw or dried plants to extraction with water or the later-described solvents after they are subjected to necessary treatments such as pulverization and heat treatment if desired, dilute solutions thereof, concentrated solutions thereof or dry powders thereof.

In case of rose fruit, loquat, burnet, bearberry, mate, mugwort, *Uncaria rhynchophylla* and cat's claw, the plant extracts for use in the invention are those extracted from parts of plants, such as leaf, stalk, bark, flower, fruit, root and stamen, whole herbage or sap.
The extracts from seaweeds are those extracted from parts or the whole of brown algae.

In case of rose fruit, the extract is particularly preferably an extract from false fruit and fruit; in case of bearberry and loquat, the extract is particularly preferably an extract from leaves; in case of burnet, the extract is particularly preferably an extract from rhizomes; in case of mugwort, the extract is particularly preferably an extract from leaves and stalks; and in case of brown algae, the extract is particularly preferably an extract from the whole thereof. The extracts from tea include not only extracts obtained by direct extraction from tealeaves collected from tea shrubs but also extracts obtained by extraction from green tea, black tea and oolong tea which are products of tea.

The plant extract of the invention has an activity of inhibiting a function of FGF-5. The activity is presumably attributable to the idea that one (or plural) kind of an ingredient supposed to be a FGF-5 inhibiting substance is contained in the extract.

### Inhibition of fibroblast growth factor 5 (FGF-5)

By virtue of the studies by the present inventors, it has been found for the first time that FGF-5 has a function of inhibiting hair growth and thereby shifting the hair cycle from the growing stage to the regression stage. On the other hand, the hair dermal papilla existing in the root of the hair follicle controls proliferation or differentiation of hair matrix cells and thereby controls the hair growth. In the regression stage, the hair matrix cells disappear, but the number of the hair dermal papilla cells is not decreased although the activity thereof is lowered. In the next growing stage, the hair dermal papilla cells are activated again, and by their actions, the hair matrix cells newly start proliferation and differentiation. The hair dermal papilla cell itself is not changed, so that a hair of the same thickness as in the previous growing stage can be produced. Therefore, if the role of FGF-5 as triggering shifting of the hair cycle from the growing stage to the regression or the resting stage is suppressed by any means, only the growing stage is prolonged. Accordingly, the substance thus prolonging the growing stage is expected to exert excellent hair growth-stimulating and hair-nourishing effects.

Actually, it has been reported by the present inventors that FGF-5S, which is a short chain molecule of the FGF-5 and occurs in a large amount in hair follicles of the growing stage, exhibits an antagonist activity against the FGF-5 and retards shifting of the hair cycle from the growing stage to the regression stage to promote hair growth. The present inventors have further studied and have found that extracts obtained from the aforesaid plants or seaweeds have an activity of inhibiting the function of FGF-5. As described later, the inhibitive action of the extracts has been confirmed by examining influences of FGF-5 or interleukin 3 (IL-3) on the cell proliferation-promoting action using cultured cells.

Elucidation of the mechanism of the FGF-5 inhibition by the extracts and the substance of the active substance must wait the results of future studies. In the extracts of the invention or a fibroblast growth factor 5 inhibitor containing one or more kinds of the extracts, one or plural substances having an antagonist activity similar to the activity of FGF-5S may be contained, or inhibiting substances directly exerting an inhibiting effect on the FGF-5 may be present.

The inhibitor of the invention is a fibroblast growth factor 5 inhibitor containing one or more kinds of extracts obtained by extraction from rose fruit, loquat, burnet, bearberry, mate, cat's claw, mugwort, *Uncaria rhynchophylla*, tea and brown algae with water or a solvent other than water. If such an inhibitor is added to a hair growth stimulant or a cosmetic, hair growth-stimulating effects are expected to exert, so that the hair growth stimulant and the cosmetic are useful.

### Preparation of plant extract

The plant used as a raw material for preparing the plant extract of the invention is particularly preferably selected from the group consisting of rose fruit, loquat, burnet, bearberry, mate, cat's claw, mugwort, *Uncaria rhynchophylla*, tea and brown algae, among the aforesaid plants.

The plants of rose fruit, loquat, burnet, bearberry, mate, cat's claw and mugwort, *Uncaria rhynchophylla* and brown algae may be subjected to extraction as they are, but taking extraction efficiency into consideration, the extraction is desirably carried out after treatments, such as thin cutting, drying and pulverization, are carried out. In case of tea, it is preferable that raw tealeaves collected from tea shrubs are used as starting materials, or tea products produced from tealeaves, such as green tea, fermented tea and semi-fermented tea, are used as starting materials. The extraction is attained by immersing the plant into an extraction solvent. In order to increase extraction efficiency, continuous or intermittent stirring may be carried out, or homogenization may be carried out in an extraction solvent. The suitable extraction temperature is in the range of usually 5°C to a temperature not higher than the boiling point of the extraction solvent. The suitable extraction time is in the range of generally 1 to 14 days although it varies depending upon the properties of a raw substance for the extraction, type of the extraction solvent, extraction temperature, etc. The extraction operation may be carried out continuously using an extraction apparatus such as a Soxhlet extractor.

The extraction method is not specifically restricted, and a usual method, such as heating extraction or ordinary temperature extraction, may be carried out. Further, the extraction may be carried out at atmospheric pressure or under pressure.

As the solvent for the extraction, not only water but also a polar organic solvent is employable. Examples of the polar organic solvents include lower alcohol type solvents, such as methanol, ethanol, propanol and isopropanol; polyhydric alcohol type solvents, such as polyethylene glycol, 1,3-butylene glycol, propylene glycol, dipropylene glycol and glycerol; ether type solvents, such as ethyl ether and propyl ether; ketone type solvents, such as acetone and ethyl methyl ketone; and ester type solvents, such as ethyl acetate and butyl acetate. A solvent having an excellent property of dissolving the substance for the extraction and having relatively low solidifying point and boiling point is preferably employed. From the above solvents, one or more solvents are selected and used for the extraction. The extraction solvent may be properly selected according to the type, part, properties, etc. of the plant used in the extraction. For example, in case of a plant containing a large amount of calcium oxalate or the like that causes skin disease, extraction alternately using plural solvents consisting of a combination of water and the organic solvent or extraction by dispensing a mixed solvent is carried out, whereby an extract from which unsuitable ingredients have been specifically removed can be obtained.

Further, a saline, a phosphate buffer solution or a phosphate-buffered saline is also employable. If necessary, a method using a fluid in a super critical state of carbon dioxide disclosed in, for example, Japanese Patent Laid-Open Publication No. 172096/1987 may be adopted.

The crude extract thus obtained can be incorporated as an active ingredient, as it is, into the inhibitor, the hair growth stimulant or the cosmetic of the invention. In consideration of the requirements for the formulation such as storage and form of the hair growth stimulant, the extract may be used after treatments, such as vacuum concentration, dilution and filtration, are further carried out appropriately. The solution extracted may be subjected to concentration, spray drying, freeze drying or the like to give a dried product.

Other operations, such as concentration, drying and re-dissolution in water or in a polar solvent, may be carried out, or within limits not detrimental to the biological actions of the extract, purifying treatments, such as decoloring, deodorizing and desalting, may be further carried out by methods well-known in the art, or purification by fractionation using column chromatography may be further carried out.

### Hair growth stimulant and cosmetic

Only by the use of a hair growth-stimulating or hair-nourishing agent containing, as an active ingredient, a hair growth-stimulating substance related to the function of the hair dermal papilla cells undertaking biological mechanism of the hair growth and based on the molecular action controlling the function, satisfactory hair growth-stimulating effects can be obtained. The hair growth stimulant of the invention having been completed from this viewpoint contains, as active ingredients, one or more kinds of the aforesaid plant extracts that inhibit the function of FGF-5. Likewise, the cosmetic of the invention contains, as active ingredients, one or more kinds of the plant extracts. The hair growth stimulant and the cosmetic of the invention are characterized in that the action of the active ingredients contained is backed up with the molecular mechanism of the hair growth and that the ingredients are derived from natural materials.

The hair growth stimulant and the cosmetic of the invention can be prepared by subjecting one or more kinds of the plant extracts selected as active ingredients and the following ingredients in given amounts to an appropriate operation such as blending in accordance with a conventional method.

The amount of the plant extract to be added, based on the total amount of the hair growth stimulant varies depending upon the type of the plant used, extraction solvent, extraction conditions, treatments after extraction, etc. The amount of the plant extract based on the total amount of a liquid hair growth stimulant is in the range of preferably 0.001 to 5.0% by weight, more preferably 0.1 to 1% by weight, with the proviso that the extract is in a state of a supernatant liquid obtained by procedures of pulverizing the plant, immersing the pulverizate in an extraction solvent and extracting. The "extract in a state of a supernatant liquid" used herein is referred to as a supernatant liquid obtained by performing extraction using 1 liter of an extraction solvent based on 100 g of a dry plant and allowing the resulting extract to stand still or a supernatant liquid obtained by centrifugation after the extraction. If the amount of the plant extract is less than 0.001% by weight, a desired hair growth promoting effect is not obtained. If the amount thereof exceeds 5% by weight, on the contrary, not only the effect reaches the uppermost limit but also the production cost is increased. In the case where the hair growth stimulant is in the form of a semisolid, such as emulsion, jelly or paste, or in the form of a fluid, the plant extract in the form of a powder or the like has only to be added in an amount converted on the basis of the amount of the supernatant liquid.

The hair growth stimulant and the cosmetic of the invention may take various formulation forms, such as liquid, emulsion, gel, cream, ointment, foam, mist and jell.

More specifically, the hair growth stimulant is provided in the form of hair tonic, hair jell, hair cream, hair treatment lotion, hair foam, hair mist, hair shampoo, hair rinse or the like.

In the hair growth stimulant and the cosmetic of the invention, general hair growth stimulant additives, e.g., moisturizers, such as oily ingredient, hyaluronic acid and ceramide, antioxidants, such as α-tocopherol and ascorbic acid derivatives, surface active agents, ultraviolet light absorbers, local stimulants, hair follicle activators, perfumes, dyes, antibacterial antifungal agents, pH adjustors, thickening agents, excipients and refrigerants, may be properly contained in an arbitrary combination within limits not detrimental to the action of the extract, in addition to the above essential ingredients. Moreover, stabilizers such as chelating agent, and percutaneous absorption accelerators may be added.

By the combined use with other hair growth promoting ingredients considered to be effective for normalization of the scalp, a synergistic action of hair growth-stimulating and hair-nourishing effects can be promoted. For this purpose, blood circulation accelerators, anti-seborrhea agents, keratolytics, etc. are employable.

The hair growth stimulant of the invention promotes hair growth irrespective of condition and constitution, improves or cures conspicuous loss of hair and exerts remarkable effects also on various alopecias. The main subject for the application of the hair growth stimulant of the invention is the hair of human head, but the hair growth stimulant can be used for improving hair of pet animals such as dog, cat, canary and macaw. Further, the hair growth stimulant of the invention can be applied to animals from which furs are collected or whose furs are used, such as sheep, Cashmere goat, alpaca, Angora rabbit, mink and fox, to promote hair growth and thereby improve gloss of furs. Thus, the hair growth stimulant of the invention contributes to improvement of qualities of furs or fur products.

The cosmetics of the invention include not only general skin cosmetics but also quasi-drugs and medicinal cosmetics, and can be used in various forms and for various purposes, such as an emulsified cosmetic of oil-in-water type or water-in-oil type, cream, cosmetic emulsion, oily cosmetic, lotion, foundation and pack.

The amount of the plant extract of the invention to be added, based on the total amount of the cosmetic varies depending upon the type of the plant used, extraction solvent, extraction conditions, treatments after extraction, etc., but the amount of the extract in a state of a supernatant liquid obtained by the extraction is determined according to the concentration of the aforesaid hair growth stimulant. That is to say, the amount of the plant extract based on the total amount of a liquid cosmetic is in the range of 0.001 to 5.0% by weight, preferably 0.1 to 1% by weight. In the case where the cosmetic is in the form of a semisolid, such as emulsion, jelly or paste, or in the form of a fluid, the plant extract in the form of a powder or the like is added in an amount converted on the basis of the amount of the supernatant liquid.

### BRIEF DESCRIPTION OF THE DRAWINGS

Fig. 1 shows inhibition actions by extracts from various plants and seaweed on the FGF-5-dependent and IL-3-dependent proliferation of FR-Ba/F3 cultured cells. On the abscissa, concentration (unit: % (V/V)) of an extract in a culture medium is plotted.
Fig. 2 shows inhibition actions by extracts from *Uncaria rhynchophylla* on the FGF-5-dependent and IL-3-dependent proliferation of FR-Ba/F3 cultured cells. On the abscissa, concentration (unit: % (V/V)) of an extract in a culture medium is plotted.
Fig. 3 shows inhibition actions by "tea extract" and "green tea extract" on the FGF-5-dependent and IL-3-dependent proliferation of FR-Ba/F3 cultured cells. On the abscissa, concentration (unit: % (V/V)) of an extract in a culture medium is plotted.

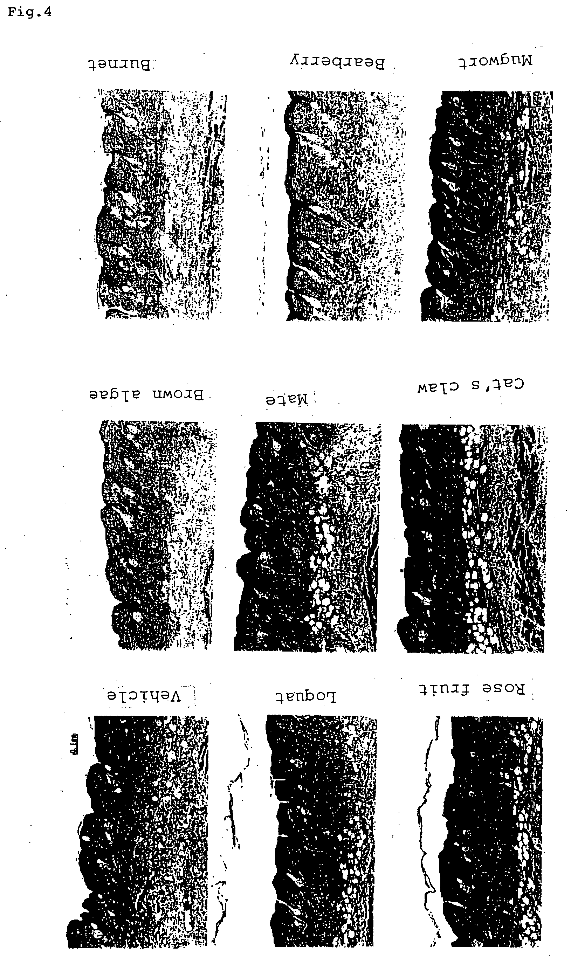

Fig. 4 is a group of photographs each showing an image of a skin slice of a mouse whose hair cycle is in the growing stage. In each photograph, the hair follicle is a structure that extends toward the epidermis from the hair bulb of saccate form existing in the dermis, and in the hair follicle a growing hair is wrapped. At the lower part of the image, subcutaneous (fat) tissue is observed. In the group wherein 50% ethanol as a vehicle was applied (the scale in this photograph indicates 0.1 mm, and the same magnification is applied to other photographs), the hair follicles were short and small and the growth of the hair follicles was still inhibited by the action of the hypodermically injected FGF-5. In contrast therewith, in the group wherein the plant extracts of the invention were applied, that is, the group of loquat, rose fruit, brown algae, mate, cat's claw, burnet, bearberry and mugwort, it is shown that the hair follicles grew toward the fat tissue and the growth of the hair follicles was recovered.
Fig. 5 is a group of photographs showing effects exerted by application of an extract from *Uncaria rhynchophylla*, "tea extract" and "green tea extract" in the same experiments as those shown in Fig. 2-1.

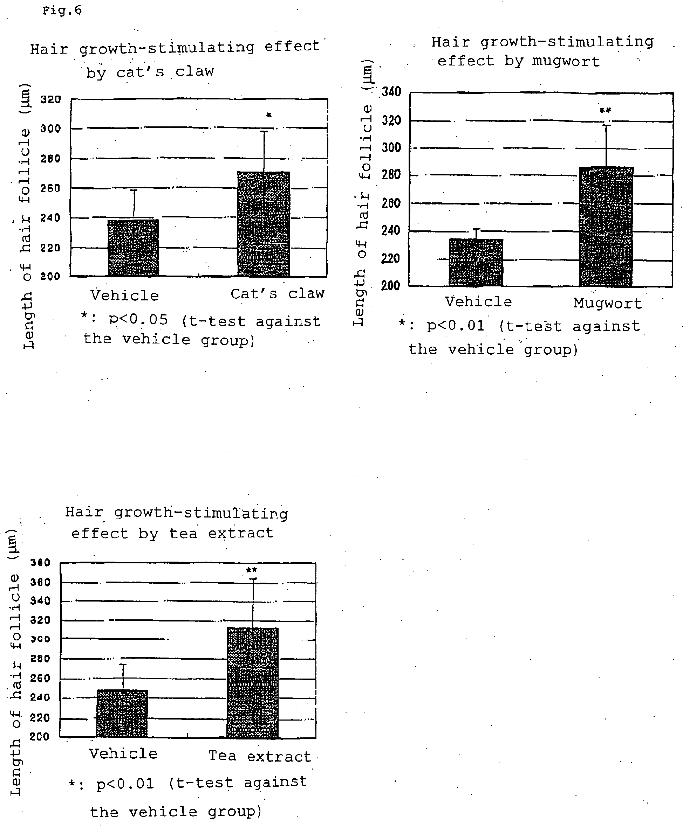

Fig. 6 shows hair growth-stimulating effects of an extract from cat's claw, an extract from mugwort and "tea extract" in the comparison between the total length of a hair follicle of a mouse to which those extracts were each applied and the total length thereof in the vehicle group.

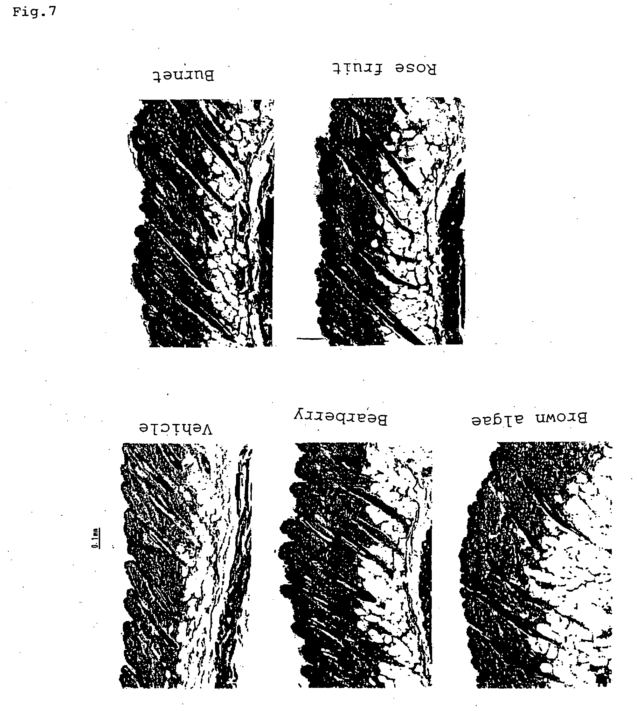

Fig. 7 is a group of photographs each showing an image of a skin slice of a mouse whose hair cycle is at the end of the growing stage. It was confirmed from the shapes and sizes of the hair follicles in the slices that in the group wherein 50% ethanol (vehicle) was applied (the scale in this photograph indicates 0.1 mm, and the same magnification is applied to other photographs), the hair follicles were shifted to be in the regression stage, while in the group wherein the plant extracts of the invention were applied, that is, the group wherein extracts from bearberry, brown algae, burnet and rose fruit were applied, the growing stage lasted and the hair follicles were maintained long.

### EXAMPLES

The present invention is further described with reference to the following examples, but it should be construed that the invention is in no way limited to those examples. In the following examples, the term "%" means "% by weight" unless otherwise noted.

### Example 1

### Preparation of plant extracts

Rose fruit (false fruit and fruit of *Rosa multiflora* Thunb. (botanical name)), leaves of loquat (botanical name: *Eriobotrya japonica Lindley*), rhizomes of burnet (botanical name: *Sanguisorba officinalis),* leaves of bearberry (botanical name: *Arctostaphylos uva-ursi(Linne)* Sprengel(Ericaceae)), leaves and stalks of mate (botanical name: *Ilex Paraguariensis*), cat's claw (botanical name: *Uncaria* tomentosa), mugwort (botanical name: *Artemisia princes)* and *Uncaria rhynchophylla* (botanical name), and whole of brown algae (Class Phaeophyceae) in each weight of 100 g on dry basis were each immersed in 1 liter of a 50% ethanol aqueous solution for 1 to 10 days at room temperature to extract soluble components. Separation of the extract from residual debris was carried out by centrifugation or filtration. The resulting supernatant liquid was used as a test material. As extracts from tea, "tea extract" (available from Tokiwa Phytochemical Co., Ltd., trade name: Tea Extract S) and "green tea extract" (available from Tokiwa Phytochemical Co., Ltd., trade name: Green Tea Extract MF) described in "Keshohin Shubetsu Haigo Seibun Kikaku (Shohaiki) (The Japanese Standards of Cosmetic Ingredients by Category)" and on the market were used after they were properly diluted.

### Example 2

### Detection of FGF-5 inhibiting substance using cultured cells

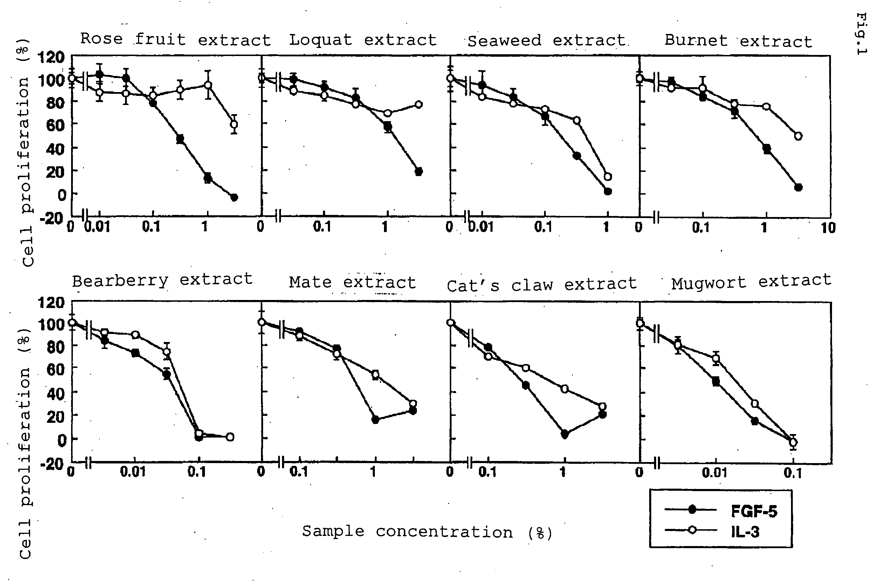

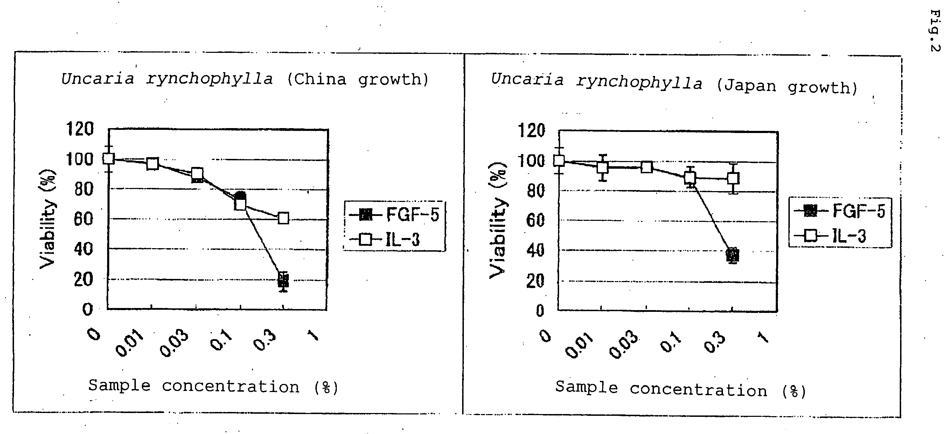

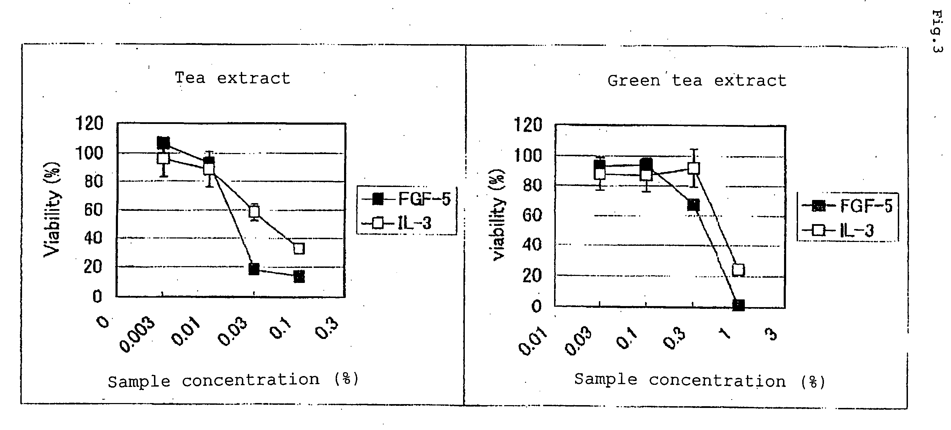

FR-Ba/F3 cells responsive to FGF-5 and interleukin-3 (IL-3) were cultured in a culture medium containing 10% fetal calf serum. IL-3 is another cytokine promoting proliferation of cells of wide range similarly to FGF-5. In a 96-well culture plate (available from Falcon), the FR-Ba/F3 cells were transplanted in a ratio of 10,000 cells/well, and then FGF-5 of 1 µg/ml or IL-3 of 10 ng/ml, a properly diluted test material (plant extract of Example 1) and 50% ethanol as a vehicle were added to the culture solution to perform cultivation for 3 days. Thereafter, a cell-counting kit (available from Wako Pure Chemical Industries, Ltd.) was added to each well, and culturing was further performed for 3 hours, followed by examination of proliferation of cells using a micro plate reader. The results are shown in Fig. 1, Fig. 2 and Fig. 3. Each of the extracts from rose fruit, loquat, burnet, bearberry, mate, cat's claw, mugwort, *Uncaria rhynchophylla* and brown algae, and "tea extract" or "green tea extract" inhibited the FGF-5-dependent proliferation of the FR-Ba/F3 cells, in lower concentrations than those in the IL-3-dependent proliferation. From these results, it is shown that the above-mentioned plant extracts selectively have a FGF-5 inhibiting activity.

### Example 3

### Experiment of application to mouse after induction of growing stage

The hair on the back of a C3H/He mouse (purchased from Japan SLC, Inc.) of an age of 8 weeks whose hair cycle was in the resting stage was pulled out to induce the growing stage. For 7 days from the day following the induction, FGF-5 of 5 µg was hypodermically injected at the same place every time, and around the injected place, the plant extracts from rose fruit, etc. (50% ethanol solutions), "tea extract" and "green tea extract" described in Example 1 were each further applied in an amount of 0.02 ml/cm² once a day. To a mouse of a control group, 50% ethanol was applied once a day in the same manner. Further, as a control to the FGF-5 hypodermic injection group, a mouse group wherein a phosphate-buffered saline solution was injected, was prepared.

On the day following the final application, the mouse was killed by dislocation of cervical vertebrae, and the skin at the applied place was taken. The skin thus taken was subjected to fixation with a 10% formalin aqueous solution, then treatment with an ethanol dehydration series, cleaning with xylene and embedding with paraffin. Using a microtome (available from Yamato), the skin was sliced in such a direction that the hair follicle from the top to the bottom could be completely visible, to prepare a skin slice of 4 µm. The resulting skin slice was extended on a silane-coated slide glass (available from Matsunami). After deparaffinizing treatment with xylene and treatment with ethanol hydrophilic series were carried out, the skin slice was dyed with hematoxylin and eosin and then subjected to treatment with an ethanol dehydration series and cleaning with xylene, followed by mounting using Canada balsam.

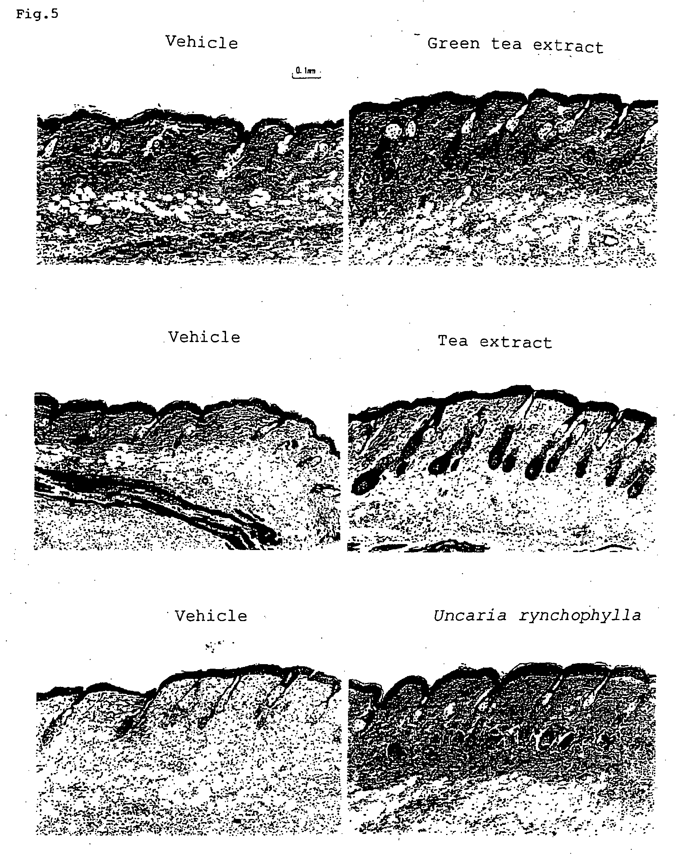

The skin slice image observed with an optical microscope was subjected to image data processing into a computer and photographing, and the shapes and sizes of the hair follicles in the slices were compared. The resulting images are shown in Fig. 4 and Fig. 5. In the group wherein FGF-5 had been hypodermically injected, growth of the hair follicles was inhibited more than that in the group wherein a phosphate-buffered saline solution had been injected (control to the FGF-5 hypodermic injection group). In the plant extract applied group, growth of the hair follicles was apparently recovered as compared with the control group wherein 50% ethanol (vehicle) had been applied. Consequently, these extracts proved to inhibit shifting of the hair cycle to the regressing stage attributable to the function of FGF-5 and proved to have a hair growth-stimulating activity.

### Example 4

### Comparison of total length of hair follicle

In order to quantitatively confirm the hair growth-stimulating activity, the plant extracts and "tea extract" described in Example 1 were each applied to a mouse having been given hypodermic injection of FGF-5, and FGF-5-injected skin slices for microscope observation were prepared in accordance with Example 3. From these slices, 50 to 100 hair follicles, the total length of each of which could be confirmed, were selected per individual, and their lengths were measured, followed by comparison with the vehicle injection group. The test was carried out using 8 mice per group. As a result, in case of the mice to which the cat's claw extract, the mugwort extract and "tea extract" have been applied, the hair follicles were significantly longer than those in the vehicle injection group.

### Example 5

### Experiment of application to mouse in the latter half of growing stage

The hair on the back of a C3H/He mouse (purchased from Japan SLC, Inc.) of an age of 8 weeks whose hair cycle was in the resting stage was pulled out to induce the growing stage. After 17 days from the induction, the hair having grown on the place from which the hair had been pulled out was removed by hair clippers, and for 3 to 4 days immediately after the removal, the plant extracts (50% ethanol solutions) described in Example 1 were each applied in an amount of 0.05 ml/cm² once a day. To a mouse of a control group, 50% ethanol was applied once a day in the same manner.

On the day following the final application, the skin of the mouse of each group was taken and a tissue slice was prepared in the same manner as described in Example 1. Then, the shapes and sizes of the hair follicles were compared. The results are shown in Fig. 7. In the ethanol (vehicle) application group as a control group, the hair follicles were short and the hair cycle had been shifted to the regression stage, while in the plant extract application group, the hair follicles were long and the growing stage still lasted. From these results, it is shown that the plant extracts of the invention prolong the growing stage of the hair cycle and apparently have a hair growth-stimulating activity.

### Example 6

### Preparation of hair tonic

55 Parts by weight of ethanol, 2 parts by weight of polyoxyethylene(8) oleyl alcohol ether, 36 parts by weight of purified water and 1 part by weight of the plant extract (supernatant liquid) obtained in Example 1 were blended. Then, a perfume such as menthol, an antioxidant such as tocopherol acetate, a preservative such as salicylic acid, a colorant and a pH adjustor were added in proper amounts in accordance with a conventional method to prepare a hair growth stimulant of hair tonic type.

### INDUSTRIAL APPLICABILITY

The hair growth stimulant and the cosmetic of the invention containing the above-mentioned extracts contain ingredients directly acting on the proliferation of hair matrix cells, so that they exerts excellent hair growth-stimulating and hair-nourishing effects and an excellent effect of preventing loss of hair. The active ingredients are extracts from plants, and therefore, the hair growth stimulant and the cosmetic of the invention can be used safely and are almost free from harmful side effects.

## Claims

1. A plant extract having an activity of inhibiting a function of fibroblast growth factor 5 (FGF-5) .

2. A plant extract obtained by extraction from a plant belonging to Rosaceae, Ericaceae, Aquifoliaceae, Rubiaceae, Compositae or Phaeophyceae with water or a solvent other than water and having an activity of inhibiting a function of fibroblast growth factor 5 (FGF 5) .

3. The plant extract as claimed in claim 2, wherein the plant is selected from the group consisting of rose fruit, loquat, burnet, bearberry, mate, cat's claw, mugwort and brown algae.

4. A plant extract obtained by extraction from *Uncaria rhynchophylla* or tea with water or a solvent other than water and having an activity of inhibiting a function of fibroblast growth factor 5 (FGF-5).

5. A fibroblast growth factor 5 (FGF-5) inhibitor containing one or more kinds of extracts obtained by extraction from rose fruit, loquat, burnet, bearberry, mate, cat's claw, mugwort, *Uncaria rhynchophylla*, tea and brown algae with water or a solvent other than water.

6. A hair growth stimulant containing as active ingredients one or more kinds of the plant extracts inhibiting a function of fibroblast growth factor 5 (FGF-5) as claimed in any one of claims 1 to 4.

7. A cosmetic containing as active ingredients one or more kinds of the plant extracts inhibiting a function of fibroblast growth factor 5 (FGF-5) as claimed in any one of claims 1 to 4.
